(19) Europäisches Patentamt
European Patent Office
Office européen des brevets

(11) Publication number: **0 389 550 B1**

(12) # EUROPEAN PATENT SPECIFICATION

(45) Date of publication of patent specification: **20.07.94**

(51) Int. Cl.5: **C07D 249/20**, D06M 13/00, D06P 1/00

(21) Application number: **89900404.8**

(22) Date of filing: **12.10.88**

(86) International application number: **PCT/US88/03547**

(87) International publication number: **WO 89/03826 (05.05.89 89/10)**

(54) **SULFONATED 2-(2'-HYDROXYARYL)-2H-BENZOTRIAZOLES AND/OR SULFONATED AROMATIC FORMALDEHYDE CONDENSATES AND THEIR USE TO IMPROVE STAIN RESISTANCE AND DYE LIGHTFASTNESS.**

(30) Priority: **21.10.87 US 111873**

(43) Date of publication of application: **03.10.90 Bulletin 90/40**

(45) Publication of the grant of the patent: **20.07.94 Bulletin 94/29**

(84) Designated Contracting States: **AT BE CH DE FR GB IT LI NL SE**

(56) References cited:
**WO-A-88/00942**
**US-A- 4 226 763**
**US-A- 4 278 589**
**US-A- 4 668 235**

(73) Proprietor: **ALLIEDSIGNAL INC.**
**101 Columbia Road,**
**P.O. Box 2245**
**Morristown, New Jersey 07962-2245(US)**

(72) Inventor: **BERENBAUM, Morris, Benjamin**
**59 Crest Drive**
**Summit, NJ 07901(US)**
Inventor: **BONFIELD, John, Henry**
**183 Lyons Drive**
**Basking Ridge, NJ 07920(US)**
Inventor: **COLE, Charles, Jayroe**
**11560 Teterling Road**
**Chester, VA 23831(US)**
Inventor: **HARRIS, Paul, Wesley**
**2321 Hey Road**
**Richmond, VA 23224(US)**
Inventor: **IZOD, Thomas, Paul**
**112 Victoria Drive**
**Basking Ridge, NJ 07920(US)**
Inventor: **ULMER, Harry, Edwards**
**Route 2, Box 3**
**Cameron, SC 29030(US)**
Inventor: **HOPF, Frederick, Robert**
**350 Parsippany Road**
**Parsippany, NJ 07054(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **YARDLEY, James, Thomas**
**40 Macculloch Avenue**
**Morristown, NJ 07960(US)**
Inventor: **BLAND, Karen, Marie**
**144 Heritage Lane**
**Hamburg, NJ 07419(US)**

(74) Representative: **Brock, Peter William et al**
**URQUHART-DYKES & LQRD**
**91 Wimpole Street**
**London W1M 8AH (GB)**

## Description

This invention relates to new sulfated 2-(2'-hydroxyaryl)-2H-benzotriazoles and their use to improve stain resistance and dye lightfastness in nylon fibers.

The prior art is replete with compositions and processes for improving the stain resistance of polyamide (nylon) fibers. The advantages of stain resistance is apparent for many of the uses of nylon, especially when used in carpets. US-A-3663157 and US-A-3519669 disclose certain formaldehyde condensation products useful as stain resists. The use of fluorine-containing agents to impart soil resistance to nylons is well known as illustrated by US-A-4414277; US-A-4209610; US-A-4195105 and US-A-4192754. US-A-3844712 (Frickenhaus) discloses a method of improving the wet fastness of polyamides dyed with cationic dyes by treatment with the salts of condensation products of formaldehyde and sulfonated diphenyl ethers. However, Frickenhaus does not disclose or recognize any improvement in stain resistance and especially, Frickenhaus does not recognize that his condensation products would increase the stain resistance of a polyamide fiber treated with a dry soil release agent.

The unsulfated precursor 2-(2'-hydroxyaryl)-2H-benzotriazoles (hereinafter sometimes called hydroxy benzotriazole or aryl benzotriazole) are disclosed for use to protect organic substances from light-induced deterioration in US-A-4226763 and US-A-4278589. Use of sulfonated 2-(2'-hydroxyaryl)-2H-benzotriazoles as photostabilizing agents for natural and synthetic fibers is disclosed in WO84/02365 and in US-A-4668235.

WO88/00942 corresponds to EP-B-0317561. It is believed to be part of the state of the art only in respect of Article 54(3) EPC. It discloses a method for improving resistance to staining by anionic staining compounds and improving lightfastness of dyes on nylon fibers which comprises treating the fiber at mild temperatures with an aqueous solution of sulfonated 2-(2'-hydroxyaryl)-2H-benzotriazole having the formula

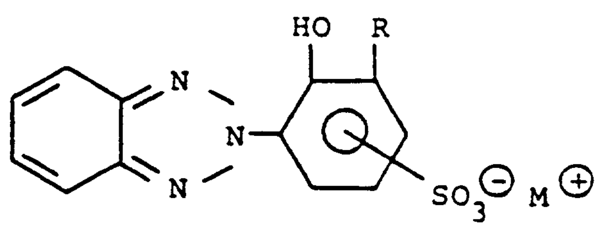

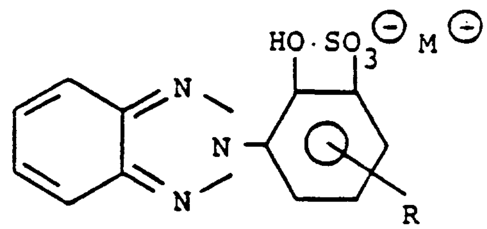

where R = tertiary alkyl or tertiary aralkyl, and M is a positive ion,

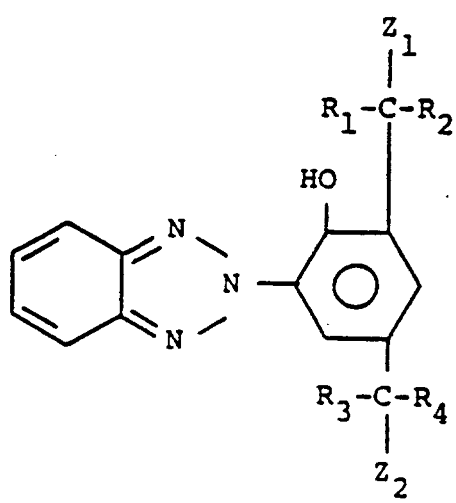

wherein $R_1$, $R_2$, $R_3$ and $R_4$ are independently the same or different alkyl groups and $Z_1$ and $Z_2$ are alkyl or sulfonated aryl group, provided at least $Z_1$ or $Z_2$ is sulfonated aryl group,
and

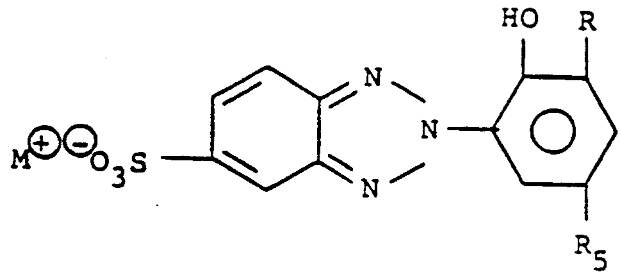

where M is a positive ion, R is tertiary alkyl or tertiary aralkyl, and $R_5$ is a short chain alkyl.

Surprisingly, it has also been found that the sulfated aryl benzotriazoles according to the invention improve resistance of synthetic nylon fibers to staining by common anionic stain agents, such as foods containing Acid Red 40 (Kool-Aid®) dye, and these sulfated aryl benzotriazoles improve lightfastness of dye on the nylon fiber. Some of these sulfated aryl benzotriazoles are new compounds, namely, compounds having the structure

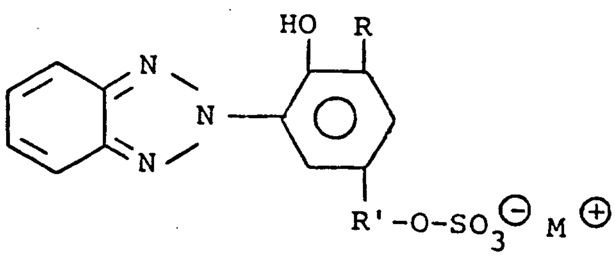

where R is a secondary alkyl or tertiary alkyl group having up to 30 carbon atoms, R' is an aliphatic chain having a total of 5 to 69 oxygen and carbon atoms in the chain, and M = hydrogen or metal, especially

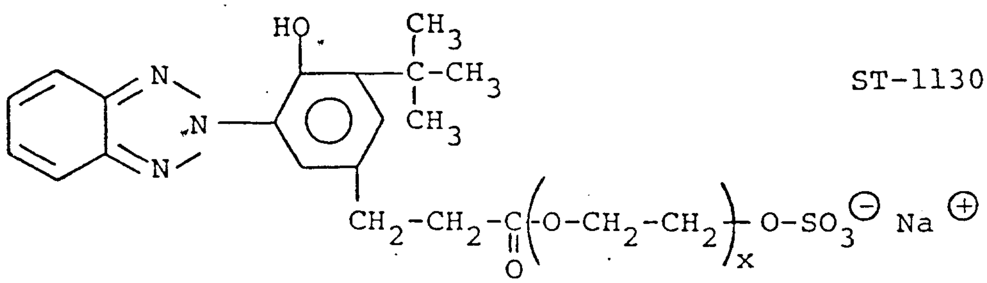

ST-1130

In these compounds, the ionic sulfate group is located at the terminus of a heteroaliphatic chain of 6 to 30 carbon and oxygen atoms, (more preferably 15 to 24 carbon and oxygen atoms). These compounds afford a surprising increase of photostability to polyamides relative to compounds VI and VII

VI

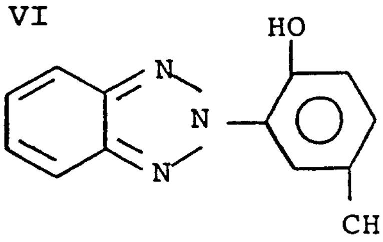

VII

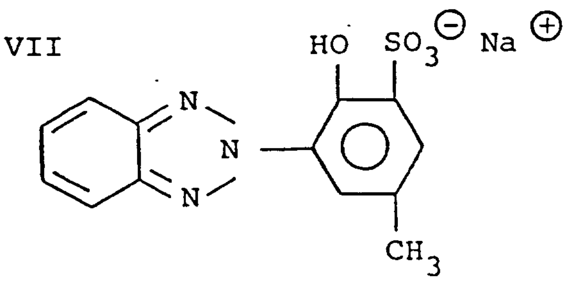

The preferred method of treatment is an aftertreatment at a pH of between 2 and 5. The preferred treatment temperature is between 30 °C and 90 °C. The preferred time to aftertreat the fiber is after dyeing,

preferably from 10 to 30 minutes in duration. The compounds are also useful to improve properties of nylon materials in other forms, such as film.

DETAILED DESCRIPTION OF THE INVENTION

When not otherwise defined, as used in this specification alkyl means a paraffin hydrocarbon radical derived from an alkane by dropping at least one hydrogen atom, such radicals containing from one to about thirty carbon atoms.

As used in this specification, aryl means an aromatic radical derived from those characteristic six-carbon ring or the condensed six-carbon ring compounds such as benzene, naphthalene, phenanthrene, anthracene, etc. derived by dropping at least one hydrogen atom therefrom.

The terms nylon and polyamide as used herein denote those synthetic long chain polyamides having recurring amide groups as an intregal part of the polymer chain. Exemplary of such polyamides are nylon 6, nylon 66, nylon 12, etc.

The aromatic compounds that may be condensed with formaldehyde for use in the process of this invention are those which contain up to 30 or more carbon atoms and preferably have at least one phenol or naphthyl group. The aromatic groups may be unsubstituted or substituted with hydroxyl, alkyl groups of 1 to 18 carbon atoms and/or fluoroalkyl of 1 to 10 carbon atoms, including prefluoroalkyl groups. The aromatic compounds may be composed of two or more aromatic rings bridged by -O-, $-SO_2$, $-C_nN_{2n}-$, -CO-, or a carbon to carbon bond. It will be understood that a necessary feature of the aromatic compound is its ability to condense with formaldehyde and to that extent a formaldehyde-reactive substitutent is necessary. Illustrative of the types of aromatic compounds that may be condensed with formaldehyde and used in the process of this invention are, benzenes, naphthalenes, xylenes, bis-phenols, phenols, naphthols, diphenyl ethers, diphenyl sulfones, diphenyl ketones, diphenyl alkanes, dinaphthyl ethers, dinaphthyl sulfones, dinaphthyl ketones, dinaphthyl sulfones, and the like. As indicated above, the aromatic compounds may be unsubstituted or substituted with hydroxyl, alkyl and/or perfluoroalkyl groups.

Preferably, the aromatic compound is first sulfonated and thereafter condensed with formaldehyde although the unsulfonated compounds may be first condensed with formaldehyde and the condensate then sulfonated. Sulfonation is preferably accomplished by direct addition of chlorosulfonic acid. Alternatively, $SO_3$ or sulfuric acid may be used although higher temperatures are required with the acid. Prior to reaction with formaldehyde, the sulfonated aromatic compound is diluted with water. An aqueous solution of formaldehyde is added with the mole ratio of sulfonated aromatic compound: Formaldehyde being in the range of 1.0 to 2.0, preferably 1.5 to 1.7.

More preferably, the sulfonated aromatic-formaldehyde condensation products are compounds having the following structure (hereinafter called the "preferred DPE condensate"):

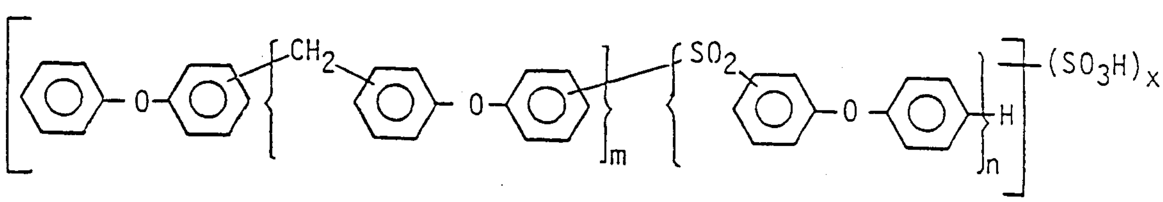

wherein m is 0 to 4, n is 0 to 3 and x is 1 to 5. Most preferred are the compounds wherein m is 0 or 1, n is 0 to 2 and x is 1 to 3, except the nonpreferred species are compounds wherein m + n = 0 or 1 along with x > m + n + 1, or wherein m = 0, n = 1 and x = 2. A particularly well-performing compound is when m = 1, n = 0 and x = 2.

Although the use of fluorinated dry soil release (DSR) agents have been previously employed to impart stain resistance to polyamide carpet fibers, it has been found in accordance with this invention, that the use of such DSR agent in conjunction with the sulfonated aromatic condensate results in a synergistic effect producing a superior stain resistance than either treatment agent alone. Additionally, the resistance of such fibers to ozone fading is surprisingly improved. The term fluorinated dry soil release agent as used herein is intended to denote those agents known and used in the art to impart increased soil resistance to polyamide fibers, particularly, carpet fibers. Particularly useful in the process of this invention are the fluorinated dry soil release agents of the types disclosed in U.S. Patent Nos. 4,191,754; 4,604,316 and 4,605,587, which are incorporated herein by reference. It is preferred that the fiber first be treated with the fluorinated dry soil

release agent and thereafter with the sulfonated aromatic-formaldehyde condensate although that sequence may be reversed.

The compounds used in the process of this invention impart excellent stain resistant properties when applied to polyamide surfaces. While not wanting to be bound by any theory, it is believed that under the special application conditions, the sulfonated aromatic-formaldehyde condensate products saturate the nylon near the surface of the fiber. This results in a high negative charge density which forms an ionic barrier and thereby inhibits the sorption of water soluble food dyes such as are found in artificially colored foods.

The sulfonated aromatic-formaldehyde condensates useful in accordance with this invention are water soluble and can be applied to nylon in a variety of ways. Typically, the compounds are applied to dyed fiber from either a dilute or concentrated aqueous solution with a concentration range between 0.001 and 75 weight percent. The solution is contacted with the polyamide fiber for 5 seconds to 45 minutes at temperatures ranging from room temperature to about 100 °C. The pH of this solution should be between 1.0 and 5.0, preferably about 2.0. It has been found that when the pH of the solution is at the lower pH values the percent exhaust and stain resist property of the treated fiber is significantly improved. For this reason, it is prefered to employ sulfonated aromatic condensates with $-SO_3H$ groups instead of sulfonate salts. After treatment, the fiber is water extracted and oven dried at 120 °C. The sulfonated condensates useful in this invention can be applied to dry or wet fiber either as a concentrate or dilute solution. The treated fibers should contain the sulfonated condensate in amounts of between 0.05 and 10% OWF (on the weight of fibers) and the treatment bath conditions should be maintained accordingly.

The method for applying the fluorinated dry soil release agent (DSR) to polyamide fibers is known in the art and is thus not necessary to describe in detail. In general, the fibers may be contacted with a spin finish containing the DSR agent in a known matter or other suitable procedures may be employed. The amount of DSR on the fibers resulting from the process of this invention should be between 0.05 and 1.0% on weight of fabric (OWF).

The treated nylon carpet fibers are tested for stain resistance in the following manner: A treated sample of nylon carpet yarn in circular knit sleeve form is placed on a non-absorbent surface and a small (10 ml) beaker containing 2 ml of staining solution e.g., Cherry Kool-Aid™ is inverted on the fabric and slowly agitated in such a manner that the liquid is retained within the beaker and in contact with the fabric until the termination of the test, or absorption of the staining solution by the fabric takes place. After 5 minutes, or other suitable period of time, the beaker and remaining solution are removed and any excess liquid on the fabric is blotted away with absorbent paper towels. The depth of the stained area is then visually assessed by use of the AATCC Gray Scale for staining, the AATCC Chromatic Transference Scale, or other suitable comparative method.

It has also been found, in accordance with this invention that certain sulfonated aromatic-formaldehyde condensates, namely those produced by reacting formaldehyde with sulfonated diphenyl ethers (DPE), can by themselves impart stain resistance to polyamide fibers. The DPE may be unsubstituted or substituted with 1-18 carbon alkyl or 1-10 carbon fluoroalkyl. These diphenyl ether condensation products are produced, as described above, by sulfonating DPE or its alkyl or fluoroalkyl derivitives and thereafter condensing with formaldehyde. Unsubstituted DPE is prefered but, prior to sulfoflation, the DPE may be alkylated or fluoralkylated. However, the alkylation process, generally conducted by a Friedel-Crafts type reaction, adds cost. Preferably the sulfonate is $-S0_3H$ as this results in a lower pH of the treatment solution. In addition, the treatment of polyamide fibers with the DPE condensate in conjunction with the above described DSR results in fiber characteristics surpassing those obtained by either agent alone.

Example 1

A diphenyl ether - formaldehyde condensation product is produced as follows: To a reactor containing 170 grams of DPE is added 128 grams of chlorosulfonic acid over a 1 hour period at 50-60 °C. After air sweeping to remove HCl, there is added 65 grams of water and thereafter, while maintaining the temperature at 50-60 °C there is added 49 grams of a 37% (by weight) aqueous formaldehyde solution over a 30-minute period. The liquid is then digested for 10 hours at 100 - 105 °C. The product 265 grams in 107 grams of water is drained from the reactor. A mixture of the "preferred DPE condensate" compounds previously described was formed. A 7.5 gram dry nylon 6 sleeve is contacted with 0.15 grams of the condensation product so produced and 175 ml of water for 30 minutes at 71 °C. The nylon 6 sleeve had been previously treated with a fluorinated dry soil release compound as described in Example 1 of U.S. Patent 4,192,754 and mock dyed. The nylon sleeve is then squeezed and paper towel dried before placing in an oven at 120 °C for 30 minutes. A 5-minute stain test with a Cherry Kool-Aid™ solution gave a 5 rating

on the Chromatic Transference Scale (5 = best, 1 = worst) compared to an untreated sleeve control rating of 1.

Example 2

A 7.5 gram nylon 6 sleeve which had not been pretreated with a fluorinated dry soil release agent but which had been mock dyed was contacted with 0.15 grams of the condensation product produced in accordance with Example 1 in 175 ml of water for 30 minutes at 71°C. The nylon sleeve is then squeezed and paper towel dried before placing in an oven at 120°C for 30 minutes. A 5-minute stain test with Cherry Kool-Aid™ solution gave a 3+ rating compared to a 1+ rating for the untreated sleeve control.

Example 3

A diphenyl ether-formaldehyde condensation product is produced as follows: to a reactor containing 170 grams DPE in 500 ml of carbon disulfide there is added 30 grams $AlCL_3$. 334 grams of hexafluoroacetone is introduced over a three-hour period while the temperature is held at 0 - 10°C. After dilution with ice water, phase separation and removal of $CS_2$, the product, a fluid liquid at 25°C, is treated with 128 grams of chlorosulfonic acid added over a one-hour period of 30 - 50°C. There is then added 80 grams $H_2O$. Aliquots are taken from the reactor and a 37 weight percent formaldehyde aqueous solution is added such that the mole ratio of sulfonated DPE to formaldehyde ranges from 1.3 to 1.8. A 7.5 gram nylon 6 sleeve which had been previously treated with a fluorinated dry soil release agent as described in Example 1 of U.S. Patent 4,192,754 is contacted with 0.15 grams of the condensation product so produced in 175 of water for 30 minutes at 71°C. The nylon 6 sleeve had been previously mock dyed. A 5-minute stain test with Cherry Kool-Aid™ solution gave a 5 rating.

Example 4

A nylon 6 sleeve which had not been pretreated with a fluorinated dry soil release agent but which had been mock dyed was contacted with 0.15 grams of the condensation product produced in accordance with Example 3 in 175 ml of water for thirty minutes at 71°C. The nylon sleeve is squeezed and paper towel dried before placing in an oven at 120°C for 30 minutes. A five-minute stain test with Cherry Kool-Aid™ solution gave a 4 rating.

Example 5

A dihydroxy diphenyl sulfone formaldehyde condensate is sold under the trade name MESITOL PS by Mobay Chemical Company. Four nylon 6 sleeves composed of fibers previously treated with the DSR described in Example 1 of U.S. Patent 4,192,754, and four nylon 6 sleeves containing no DSR, are contacted with a solution of the sulfonated aromatic condensate under conditions adjusted to result in treated sleeves containing 0.2%, 0.5%, 1.0% and 2.0%, OWF of the condensate. Stain testing of the resulting samples, along with two control samples, as described in Example 1, gave the following results:

| % OWF | No DSR | DSR Treated |
|---|---|---|
| 0 | 1 | 2 |
| 0.2 | 1 | 3 |
| 0.5 | 2 | 4 |
| 1.0 | 2.5 | 5 |
| 2.0 | 3.5 | 5 |

An additional feature of the present invention lies in a process for treating polyamide fibers with the sulfonated aromatic-formaldehyde condensates described above in conjunction with treatment of the fibers with 0.1 to 5.0% (OWF) of the 2-(2-hydroxyaryl)-2H-benzotriazoles described in Examples 8 to 15. Surprisingly, the treatment with these benzotriazoles does not adversely affect the stain resistance characteristics obtained with the sulfonated aromatic-formaldehyde condensate while the lightfastness of the treated fibers exhibits significant improvement.

Example 6

Nylon 6 sleeves predyed silver-gray as in Example 13 were treated with aqueous baths containing a condensate of formaldehyde and unsubstituted diphenyl ether prepared as described in Example 1 above, and the compounds described in Example 8 (Example 6a) and Example 9 (Example 6b). The bath temperature was 110°F (38°C) and pH 2.1. Similarly a control was prepared from a bath containing only the sulfonated DPE-formaldehyde condensate of Example 1 (Example 6c). Bath conditions and treatment time were such that the treated nylon contained 2.0% OWF of the DPE-formaldehyde concentrate and 0.5% OWF of the benzotriazole. After treatment stains were created on the sleeves with Cherry Kool-Aid™ containing FD & C Red 40 by forcing about 5 cc of the Kool-Aid™ into the fabric of the sleeve and blotting after 5 minutes. Results are given below. Stain rating is on an 0 to 10 scale used by trained observers, unaware of which sample was treated with which agent(s). In this scale 0 is best and 10 is worst. The lightfastness was measured by AATCC 16E.

| | Stain Rating | Lightfastness | |
|---|---|---|---|
| | | ΔE 120 AFU | Gray Scale* 120 AFU |
| 6a | 0.75 | 2.52 | 3.67 |
| 6b | 0.75 | 1.70 | 3.67 |
| 6c | 0.75 | 3.49 | 3.17 |

* Average

Improved lightfastness, i.e., less fading, is reflected by higher Gray Scale average and lower ΔE.

Example 7

The effect of the process of this invention on the ozone fading of polyamides is illustrated as follows: Samples of nylon 6 carpet, which had been treated with 2% OWF of a diphenyl ether-formaldehyde condensation product in the manner described in Example 1 except that the treatment bath temperatures were set at 110°F (38°C), 120°F (49°C), and 140°F (60°C) were treated for ozone fading by AATCC Method 129 (2 cycles) with the following results:

| Bath Temperature | 110°F (38°C) | 120°F (49°C) | 140°F (60°C) |
|---|---|---|---|
| Control*, Gray Scale Reading | 1 - 2 | 2 | 2 |
| Example 7, Gray Scale Reading | 3 | 2 - 3 | 3 |

* The control sample was treated only with DSR.

This invention relates to the preparation of novel chemical compounds and to novel methods of application to nylon (polyamide) materials (particularly polyamide fibers) which may or may not contain dyes wherein (a) the nylon materials and any incorporated dyes are afforded significant reduction in photodegradation and/or (b) the nylon materials are afforded significant improvement in resistance to many common stains, especially stains involving anionic species (such as FD & C Red 40, a common foodstuff dye). The novelty of the invention lies in (a) the chemical composition of certain of the additives which have not been previously reported and which provide significant and surprising photostability to polyamides and to dyes in polyamides at very low loadings relative to traditional photostabilizing additives, and (b) the method of application which provides for effective and durable incorporation into polyamides from aqueous solution under mild temperature conditions.

Traditional agents employed to enhance photostability of dyed polyamides include compounds of structures such as

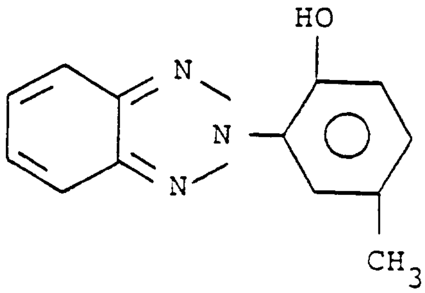

Tinuvin® P

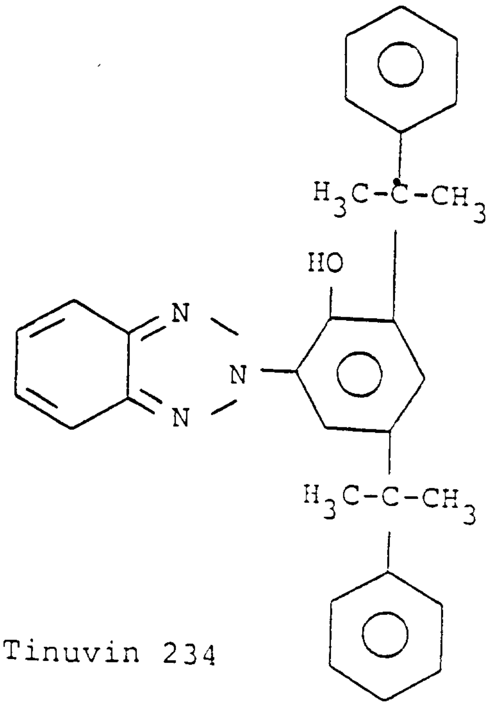

Tinuvin 234

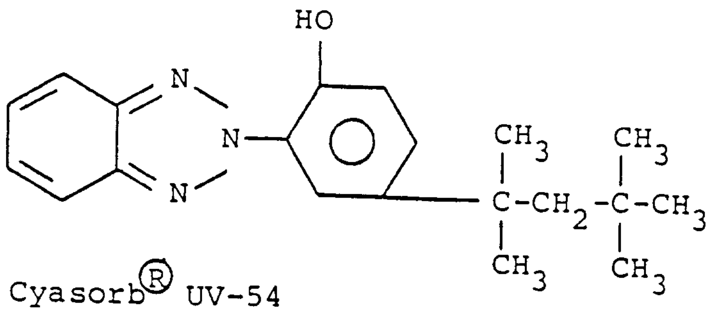

Cyasorb® UV-54

Since these compounds are generally soluble only in organic solvents, the incorporation of such materials into nylon poses serious problems. Surface application from organic solvents is a hazardous process and may result in a non-uniform coating of poor durability. Addition of these materials during extrusion may result in thermal degradation, ineffective distribution of material, and loss of material due to volatility under extruder conditions.

We have found that compounds of the general structure

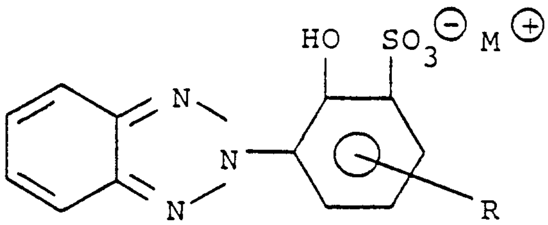

where M is a positive cation, such as an alkali metal or hydrogen, R is secondary alkyl, tertiary alkyl, tertiary aralkyl or hydrogen, for example,

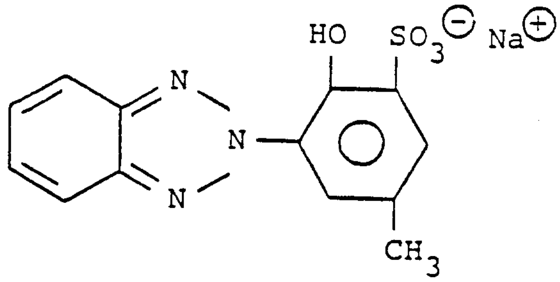

STP

may be effectively incorporated into nylon fiber from aqueous solution at relatively low pH (preferably pH < 3) and at modest temperatures (0°C-100°C, preferably 20-70°C). Nylon fibers or dyed nylon fibers treated with this material (0.05-5 percent, preferably 0.1-1 percent by weight) either before or after dyeing show dramatically enhanced photostability relative to untreated materials. In addition, these fibers demonstrate increased resistance to staining by certain common stains, especially those containing anionic groups.

In addition, we have discovered that compounds of the general structures

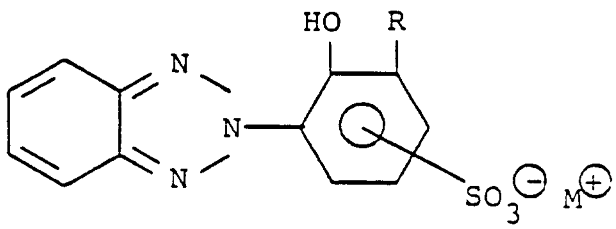

where R = secondary alkyl, tertiary alkyl or tertiary aralkyl, and particularly sulfonated T-234 (ST-234), which is a mixture of

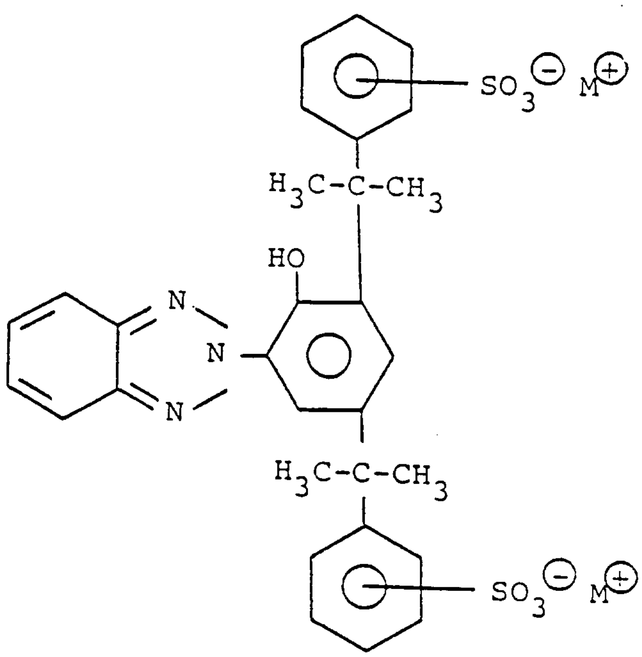

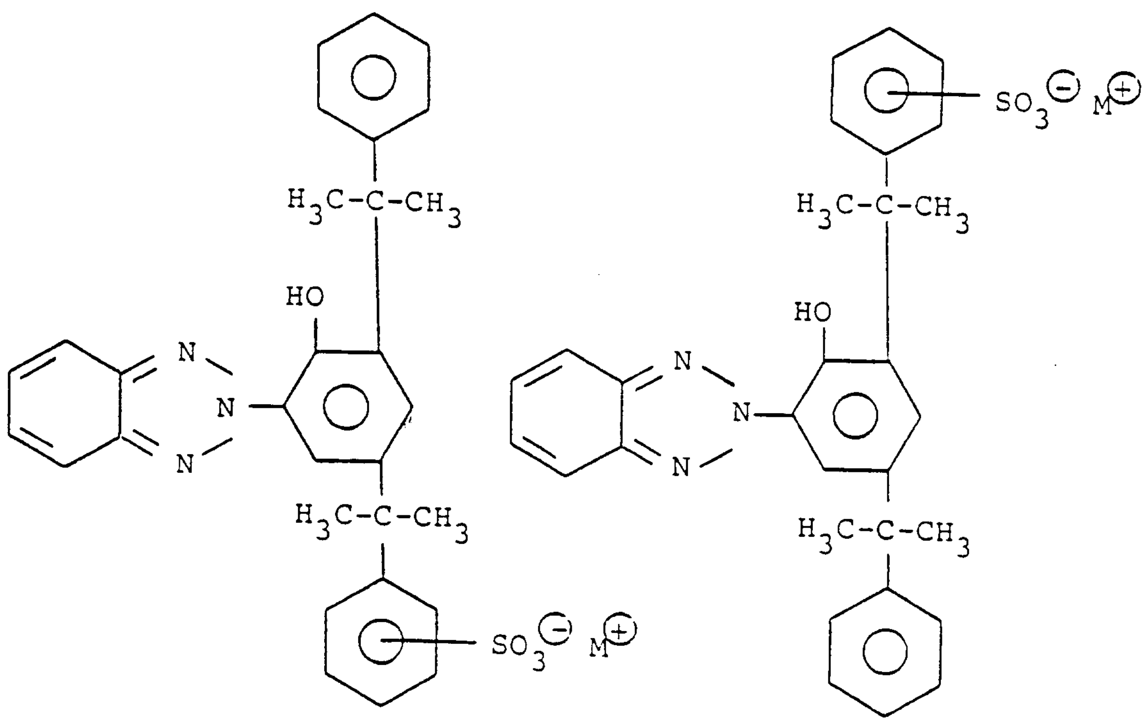

where M is a metal or hydrogen, and the $SO_3^{\ominus}M^{\oplus}$ group is para or ortho afford a surprising increase of stain resistance of nylon fiber and lightfastness of dye on nylon fiber.

Example 8

Synthesis of Sulfonated Tinuvin P or STP.

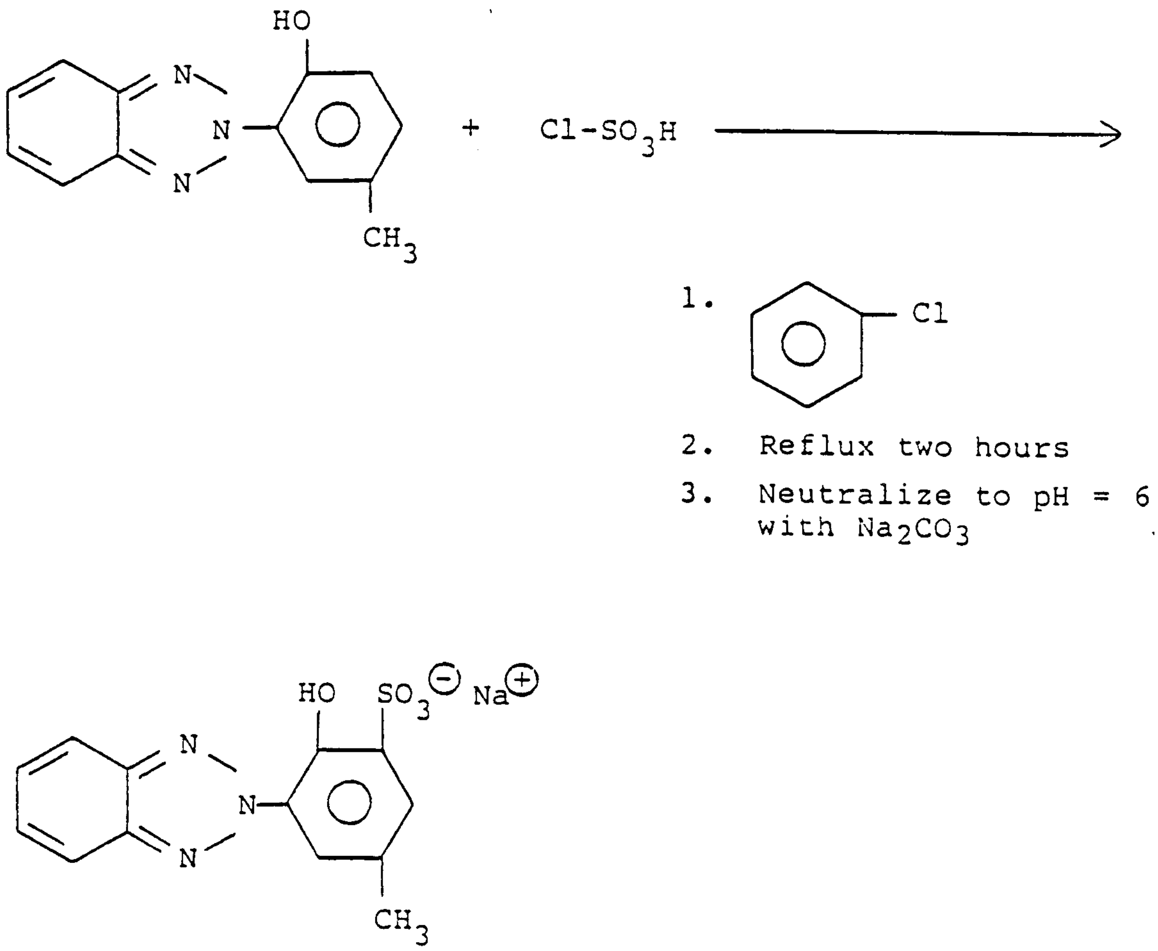


A solution of 53.5 grams (0.238 mole) of Tinuvin-P in 650 milliliters of chlorobenzene was added to a 1 liter round bottom one-neck flask equipped with a magnetic stirrer and heating mantle and set up for reflux under an atmosphere of nitrogen. Then 15.85 milliliters (0.238 mole) of reagent grade chlorosulfonic acid was slowly added to the stirred solution over a period of approximately twenty minutes. The resulting mixture was brought to reflux and maintained at reflux for 1-$^1/_2$ hours. The solution was allowed to cool to room temperature under a slow stream of nitrogen. The contents of the flask were poured, with stirring, into a 2-liter beaker containing 300 grams of crushed ice.

The resulting emulsion was neutralized to pH = 6.0 with 10 percent aqueous sodium carbonate solution. The solid product was filtered on a large Buchner funnel, using a medium speed filter paper. After several hours of air drying on the funnel, the resulting precipitate was washed with three 250-milliliter portions of toluene. The washed product was air-dried for ten hours. This nearly dry crude product was recrystallized from 4.5 liters of boiling water which had a pH = 5 (adjusted with sulfuric acid). The precipitate was allowed to settle at room temperature for 12 hours, then cooled to approximately 5°C in a refrigerator for three hours.

The resulting, finely divided, nearly colorless crystals were filtered on a coarse fritted glass funnel, and air dried for several hours. Actual yield of dry product was 51.6 grams, which is 71.2 percent of the theoretical yield.

Example 9

Synthesis of ST-234

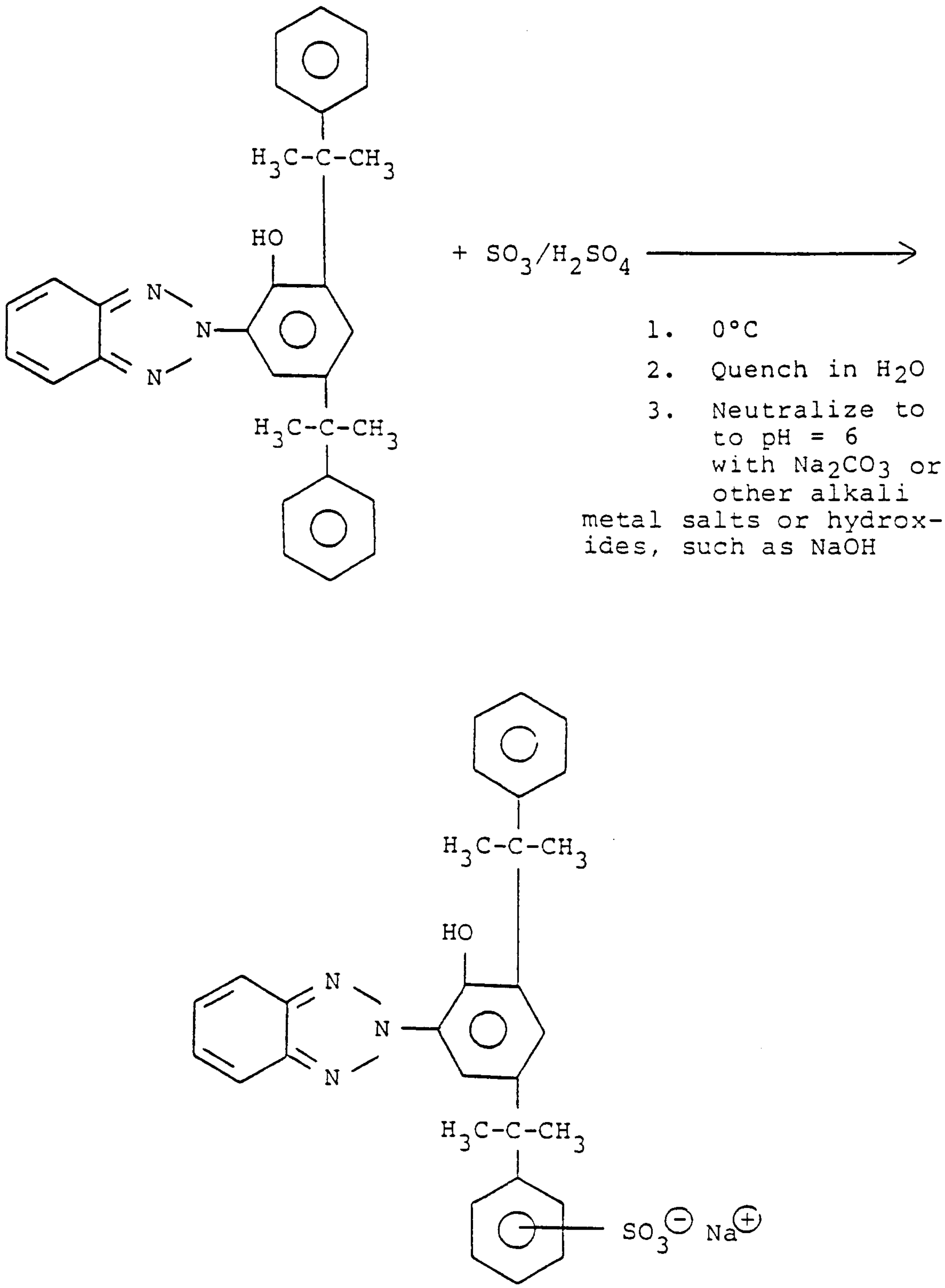


A solution of 5 percent fuming sulfuric acid was prepared by adding 2.3 milliliters of 20 percent fuming sulfuric acid to 6.8 milliliters of concentrated sulfuric acid (0.0105 mole of $SO_3$). Then 4.47 grams (0.1000 mole) of powdered Tinuvin-234 were slowly added to the vigorously stirred solution of fuming sulfuric acid

which was maintained at a temperature below 25 °C. Most of the Tinuvin-234 went into solution. The small residue of undissolved solid was allowed to stir at room temperature for about one hour until complete dissolution.

The yellow reaction mixture was quenched in 50 milliliters of ice water. The resultant mixture was neutralized to pH = 7 with saturated aqueous sodium carbonate solution. The resultant total volume was approximately 150 milliliters. This mixture was allowed to stand and settle for 12 hours. The precipitate was collected on a coarse glass fritted disc filter and washed three times with 20-milliliter portions of cold water. The precipitate was vacuum dried at 50 °C for 12 hours. The weight of dried product is nearly the theoretical amount, but contains a small percentage of coprecipitated sodium sulfate. This material was used to treat nylon for improved lightfastness without further purification.

Example 10.

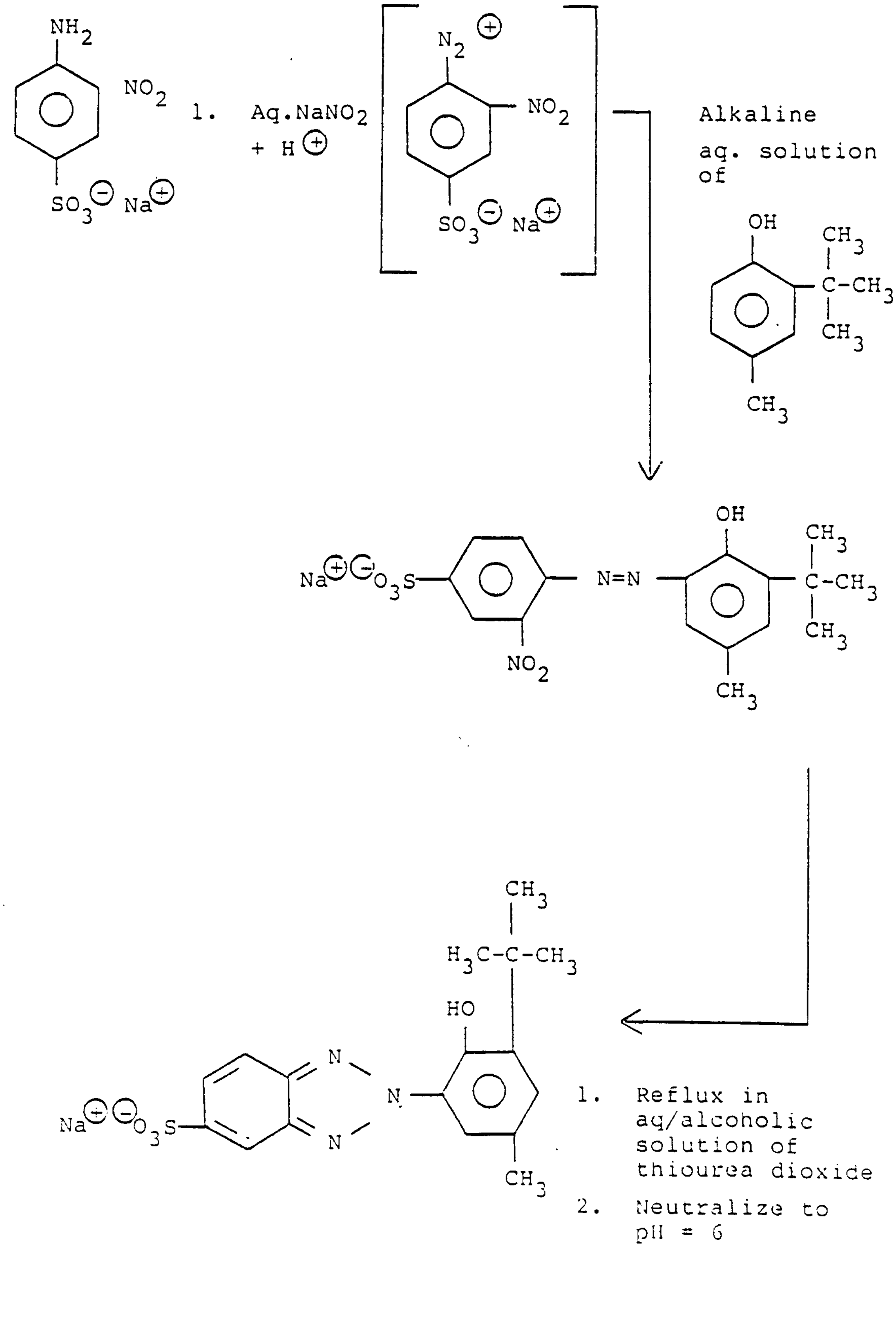
1. Aq.NaNO2
+ H⊕
Alkaline
aq. solution
of
1. Reflux in
aq/alcoholic
solution of
thiourea dioxide
2. Neutralize to
pH = 6

Example 11

Photostability of UV Light Screens Dissolved in Polymer Film Matrices

| Light Screen Agent | Polymer | Destruction, % * |
|---|---|---|
| Tinuvin P | PMMA ** | < 9 |
| Tinuvin P | Nylon-6 | >65 |
| STP | PMMA | < 9 |
| STP | Nylon-6 | >60 |
| Tinuvin 234 | PMMA | < 7 |
| Tinuvin 234 | Nylon-6 | < 5 |
| ST-234 | Nylon-6 | < 5 |

* Twenty-one hours of irradiation in a Rayonet Photochemical Reactor equipped with RPR-3000 lamps at temperatures of 38 - 40 °C and ambient humidity. Amount of destruction determined by ultraviolet spectrophotometry.
** Polymethylmethacrylate.

This example demonstrates that hydroxybenzotriazoles lose photostability in a polyamide environment. It also demonstrates that incorporation of bulky hydrophobic groups near the intramolecular hydrogen bond (especially in the ortho position relative to the hydroxyl) effectively provides for a high degree of photostability.

Typical Application Procedure

Beakers containing from 0.5 to 2.0 percent (OWF) of STP or ST-234 or other soluble UV screen with a 20:1 liquid ratio and adjusted to the proper pH were heated to 71 °C in a water bath (water bath had equilibrated at 71 °C before adding "dye" beakers). Samples of nylon knitted sleeve were added (usually 1 or 2 samples, each weighing approximately 5 grams) and stirred constantly for 30 minutes. The sleeves were removed after 30 minutes and rinsed with distilled water. After rinsing, the sleeves were padded with paper towels to remove excess water. The damp sleeves were then placed in an oven (~100 °C) for one hour. The dried samples were then allowed to equilibrate under ambient conditions, in the dark for at least 12 hours before irradiation testing.

Irradiation

Samples were suspended in a Rayonet RPR-100 Photochemical Reactor manufactured by The Southern New England Ultraviolet Company. For all of the irradiation testing reported, the reactor was fitted with 16 RPR-3000 lamps. The major output of these lamps is centered at 300 nanometers, with a significant 254 nanometers component and small amounts of radiation of longer wavelengths. No attempts were made to filter the output or restrict exposure to a specific bandwidth. During all irradiations, the internal air circulation fan was operating. This resulted in operating temperatures of approximately 48 to 52 °C. No attempt was made to control humidity.

The samples were suspended at the midpoint of the sources and rotated on a turnable to assure uniformity of irradiation. To further assure uniformity of exposure, the samples were rotated equally between direct front and back surface exposure. On longer term irradiations, the samples were cycled several times between ambient dark conditions and ambient irradiation conditions.

Example 12

Protective Effects of Water-Soluble UV Light Screens on Undyed Nylon-6 Knitted Sleeves

| Additive* | pH | Exhaustion, % ** | Color Before Irradiation | Irradiation*** |
|---|---|---|---|---|
| Control | - | - | White | Yellow |
| STP | 2 | >95 | White | Light Yellow |
| STP-234 | 2 | >95 | White | Slightly Yellow |
| STP | 5 | 25 | Light Yellow | Yellow |
| STP-234 | 5 | 35 | White | Slightly Yellow |

* Applied at 71 °C from 20:1 liquor ratio bath. Nominally loading 0.1 - 2.0%.
** Percent bath exhaustion.
*** Irradiation for 12 hours and for 40 hours in Rayonet Photochemical Reactor with RPR-3000 Lamps at 38 - 40 °C temperature and ambient humidity.

This example illustrates that uptake and color of nylon sleeves treated with sulfonated UV light screens is vastly superior for application at pH = 2 relative to application at pH = 5. Also, the superior performance of ST 234 to STP is demonstrated.

Example 13

Protective Effects of Water-Soluble UV Light Screens on Dyed* Nylon-6 Knitted Sleeves

| Additive** | pH | Uptake,% *** | Color Before Irradiation | Color After Irradiation**** |
|---|---|---|---|---|
| Control | - | - | Normal | Severely Faded |
| STP | 2 | >95 | Slightly Off-Shade | Good Protection |
| ST-234 | 2 | >95 | Normal | Excellent Protection |
| STP | 5 | 25 | Off-Shade Yellow Coloration | Good Protection |
| ST-234 | 5 | 40 | Normal | Excellent Protection |

* Grey dyeing was with the following dyes at the following conditions.

OWF, % (1)

0.0115 Tectilon Orange 3G (100% strength) (C.I. Acid Orange 156)

0.0121 Tectilon Red 2B (100% strength) (C.I. Acid Red 361)

0.0135 Telon Blue BRL (200% strength) (C.I. Acid Blue 234)

(1) On weight of fabric

Dyeing Conditions:

1% Dowfax 2A1

2% monosodium phosphate

pH 7 adj. w/trisodium phosphate

Boil 30 minutes

** Applied at 71°C from 20:1 liquor ratio, 0.1 - 2.0% (OWF) loading on non-DSR knitted sleeves. Similar results are obtained at room temperature.

*** Percentage bath exhaustion, by spectrophotometric determination.

**** After 12 hours irradiation in a Rayonet Photochemical Reactor with RYR-3000 lamps at temperatures of 38 - 40°C and ambient humidity.

This example demonstrates that UV screens which are sulfonated and which possess a bulky hydrophobic group near the intra-molecular H-bond (such as ST-234) may be effectively applied to dyed nylon sleeves and that such materials provide superior light screening capability compared to similar compounds without such a hydrophobic group (i.e., STP).

BEST MODE

The compounds useful for this invention can be applied as an aftertreatment to dyed fibers, such as carpet face fibers, the preferred compounds are the ones labeled ST-234 above. The preferred method is described in Examples 6 and 15. In use a mixture of mono- and disulfonated, both para and ortho isomers, are used. The compound may be applied in the aftertreatment either alone or in combination with other compounds, particularly those compounds which enhance stain resistance of the fibers, such as condensation product of formaldehyde with a diphenyl ether (hereinafter called DPE condensate), as described in Example 15. DPE condensate is described above in Example 1 to 7.

The recommended aftertreatment conditions for ST-234 are: 0.1 to 0.5 percent on weight of fabric (OWF) concentration in the aftertreatment bath, pH 2.1 (with citric acid), bath temperature 140°F (60°C), liquor to goods ratio 25:1, fabric time in bath 20 minutes.

Example 14

The mixture of compounds labeled ST-234 above was used in an aftertreatment bath to treat nylon carpet fiber previously dyed to a silver-gray commercial carpet fiber color. Bath conditions were as set forth above except pH was 3.0, liquor to goods ratio was 30:1 and 0.47 percent (OWF) was applied. Lightfastness was improved over the control (no aftertreatment) to 1.45 E from 4.12 E for the control, or to an average Gray Scale rating of 3.5 at 120 SFU compared to control at 1.33 Gray Scale rating.

Silver dyeing was with the following dyes at the following conditions.

OWF, % (1)

0.0104 Tectilon Orange 3G (100% strength) (C.I. Acid Orange 156)
0.0054 Telon Red BRCL (250% strength) proprietary (Mobay)
0.0126 Telon Blue BRL (200% strength) (C.I. Acid Blue 324)

(1) On weight of fabric

Dyeing Conditions:

1% Dowfax 2Al
2% monosodium phosphate
pH 7 adj. w/trisodium phosphate
Boil 30 minutes

Example 15

Staining Improvement

Using the recommended aftertreatment bath conditions given above, except temperature was 110°F (43°C), the mixture of compounds labeled ST-234 above, or the compound labeled STP above, either alone

or with the stain resistance enhancer FDE described above, were added to the aftertreatment bath for nylon carpet fiber in circular knitted sleeve form, predyed to the silver color described above. After treatment, stains were created with Cherry Kool Aid®, containing FD & C 40 food coloring, by forcing about 5 cubic centimeters of Cherry Kool Aid® into the fabric of the knitted sleeve of carpet fiber and blotting after five minutes. Results are given in the table below. Stain rating is on a 0 to 10 scale used by trained observers, unaware of which carpet fiber sample sleeve was treated with which compound. In the scale, low numbers mean good stain resistance and vice versa.

| Additive | OWF, % | Lightfastness | | Stain Rating |
|---|---|---|---|---|
| | | ΔE 120 AFU | Gray Scale* 120 AFU | |
| None (Control) | 0 | 4.12 | 1.33 | 5 |
| FDE/STP | 2/0.5 | 2.52 | 3.67 | 0.75 |
| FDE/ST-234 | 2/0.5 | 1.70 | 3.67 | 0.75 |
| FDE | 2 | 3.49 | 3.17 | 0.75 |
| STP | 0.5 | 2.47 | 3.17 | 3.5 |
| ST-234 | 0.5 | 2.35 | 3.00 | 3.5 |

* Average, by AATCC 16E

Thus, it can be seen that STP and ST-234 compound improved the stain resistance considerably over the control, but that best stain resistance is dependent on use of DPE, with or without STP or ST-234 compounds. However, the best overall combination of properties considering both lightfastness and stain resistance is the combination of DPE condensate with ST-234 which had an ΔE rating of only 1.7, and average Gray Scale reading of 3.67. Low ΔE numbers and high Gray Scale numbers mean less fading.

The use of the compounds of the present invention are preferentially concentrated near the surface of e.g., fibers of nylon to ensure optimum effective absorption of wave lengths of radiation which cause photo degration of nylon and dyes in nylon. The anionic portion of these molecules ensures that they will be strongly attracted to nylon under the application conditions. However, the relatively hydrophobic remainder of the molecules will not readily migrate into the bulk of nylon. This unique balancing of effects results in optimal near-surface concentration of these light screens. This renders them remarkably effective both as UV light screens and as stain repelling agents.

We have discovered that compounds of the general structure

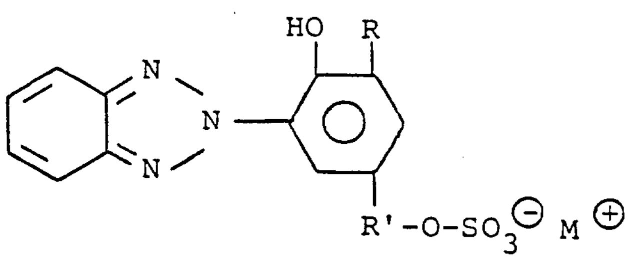

where R is secondary alkyl, tertiary alkyl group, and M = hydrogen or metal, especially

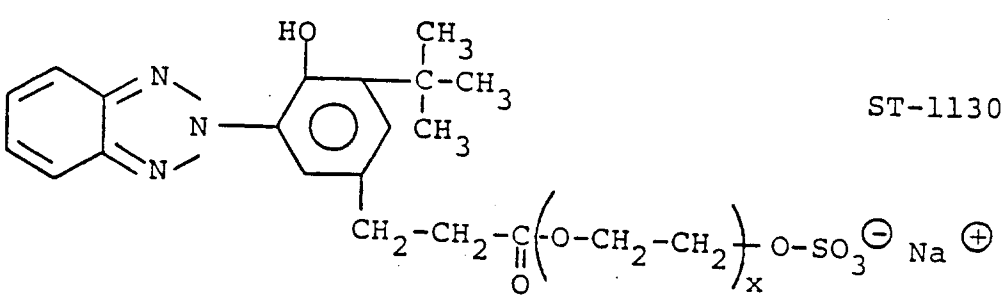

ST-1130

wherein the ionic sulfate group is located at the terminus of a heteroaliphatic chain of 6 to 30 atoms (preferably about 15 to 24 atoms) afford a suprising increase of photostability to polyamides relative to compounds VI and VII.

Example 16

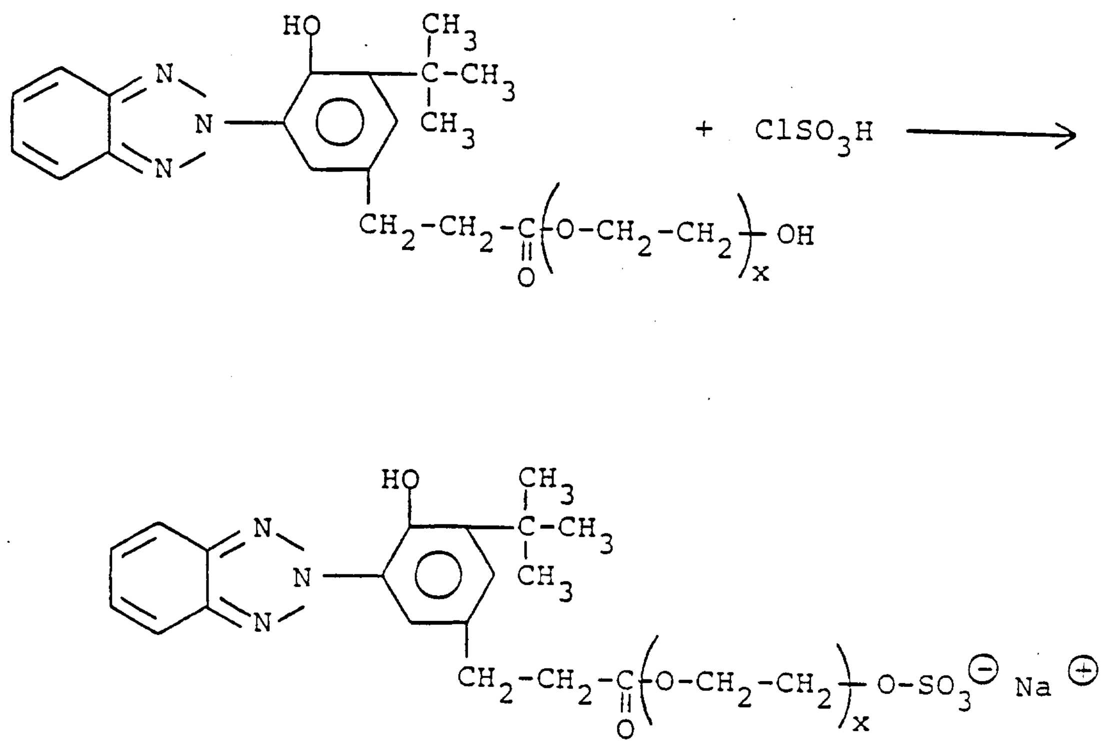

where x ≈ 6-7

1.

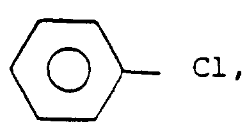

R.T.

2. Neutralize to pH = 6 with $Na_2CO_3$ or other base

3. Evaporate solvent to dryness

The same procedure can also be used when X = 8-9 or 12-13 or the entire range of 6-30.

Example 17
(Prospective example)

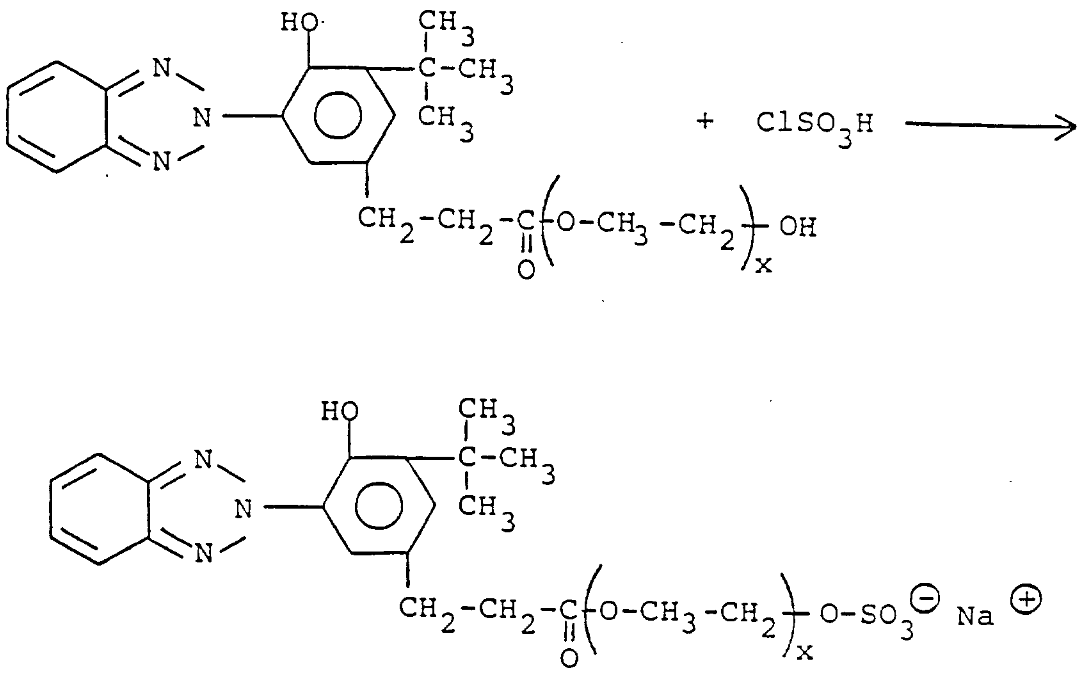


where x ≈ 6-8

1.

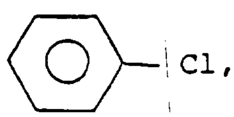

R.T.

2. Neutralize to pH = 6

3. Remove solvent

The same procedure can also be used when x = 16-22 or the entire range of 6-30.

Example 18

Photostability of UV Light Screens Dissolved in Polymer Film Matrices.

| Light Screen Agent | Polymer | Destruction, * % |
|---|---|---|
| Tinuvin P | PMMA | < 9 |
| Tinuvin P | Nylon-6 | >65 |
| STP | PMMA | < 9 |
| STP | Nylon-6 | >60 |
| Tinuvin 1130 | PMMA | <13 |
| Tinuvin 1130 | Nyon-6 | < 5 |
| ST-1130 | PMMA | < 5 |
| ST-1130 | Nylon-6 | < 5 |

* Twenty-one hours of irradiation in a Rayonet Photochemical Reactor equipped with RPR-3000 lamps at temperatures of 46-52 °C and ambient humidity. Amount of destruction determined by ultraviolet spectrophotometry.

This example demonstrates that hydroxybenzotriazoles lose photostability in a polyamide environment. It also demonstrates that incorporation of bulky hydrophobic groups near the intramolecular hydrogen bond (especially in the ortho position relative to the hydroxyl group) effectively provides for a high degree of photostability.

Example 19

Protective Effects of Water-Soluble UV Light Screens on Undyed Nylon-6 Knitted Sleeves.

| Additive[1] | pH | %[2] | Irradiation | Irradiation[3] |
|---|---|---|---|---|
| Control | - | - | White | Yellow |
| STP | 2 | >95 | White | Light Yellow |
| ST-1130 | 2 | >95 | White | Slightly Yellow |
| STP | 5 | 25 | Light Yellow | Yellow |
| ST-1130 | 5 | 55 | White | Slightly Yellow |

[1]Applied at 71 °C from 20:1 liquor ratio bath. Nominal loading o.1-2.0%.
[2] Percent bath exhaustion.
[3] Irradiation for 12 hours in a Rayonet Photochemical Reactor with RPR-3000 Lamps at 46-52 °C temperature and ambient humidity.

This example illustrates that uptake and color of nylon sleeves treated with anionic, sulfated UV light screens is vastly superior for application at pH = 2 relative to application at pH = 5. Also, the superior performance of ST 1130 relative to STP is demonstrated.

Example 20

Protective Effects of Water-Soluble UV Light Screens on Dyed[1] Nylon-6 Knitted Sleeves.

| Additive[2] | pH | Exhaustion, % [2] | Color Before Irradiation | Color After Irradiation[4] |
|---|---|---|---|---|
| Control | - | | Normal | Severly Faded |
| STP | 2 | >95 | Slightly Off-shade | Good Protection |
| ST-1130 | 2 | >95 | Normal | Excellent Protection |
| STP | 5 | 25 | Off-shade Yellow Coloration | Good Protection |
| ST-1130 | 5 | 55 | Normal | Excellent Protection |

[1] Dyed with 0.1% (OWF) argent grey

[2] Applied at 71 ° C from 20:1 liquor ratio, 0.1-2% (OWF) loading on non-DSR knitted sleeves.

[3] Percentage bath exhaustion, by spectrophotometric determination.

[4] After 12 hours irradation in a Rayonet Photochemical Reactor with RPR-3000 lamps at temperatures of 48-52 ° C and ambient humidity.

This example demonstrates that UV screens which are rendered anionic by sulfation and which posses a bulky hydrophobic group near the intramolecular H-bond (such as ST-1130) may be effectively applied to dyed nylon sleeves and that such materials provide superior light screening capability compared to similar compounds without such a hydrophobic group (i.e., STP).

## Claims

**1.** A sulfated 2-(2'-hydroxyaryl)-2H-benzotriazole having the formula:

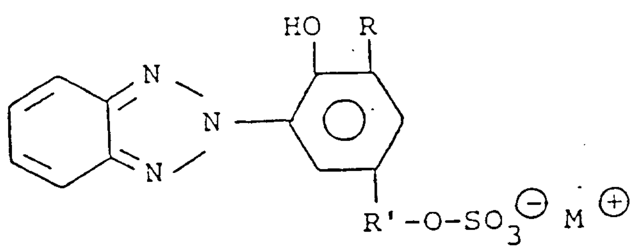

wherein R is secondary alkyl or tertiary alkyl group having up to 30 carbon atoms, R' is an aliphatic chain having a total of 5 to 69 carbon and oxygen atoms in the chain and M is hydrogen or a metal ion.

**2.** The benzotriazole of Claim 1 having the formula:

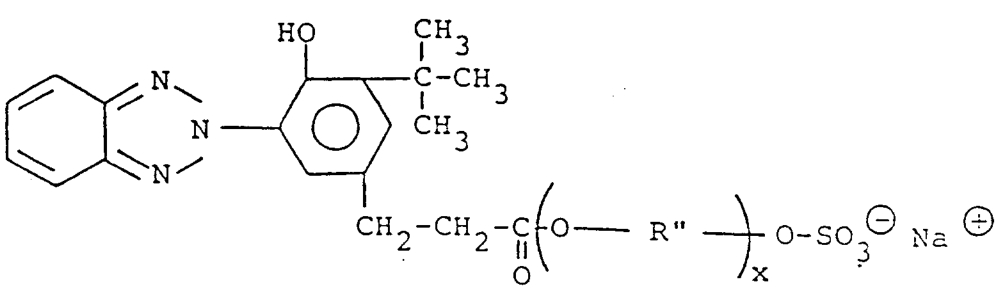

wherein x is 1 to 22 and R'' is $-CH_2CH_2-$ or

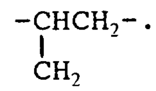

3. A method for improving resistance to staining by anionic staining compounds and improving lightfastness of dyes on nylon material which comprises treating the fiber at mild temperatures with an effective amount of an aqueous solution of sulfated 2-(2'-hydroxyaryl)-2H-benzotriazole having the formula:

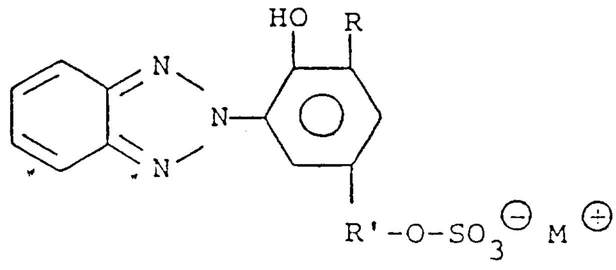

wherein R is secondary alkyl or tertiary alkyl group having up to 30 carbon atoms, R' is an aliphatic chain having a total of 5 to 69 carbon and oxygen atoms in the chain and M is hydrogen or a metal ion.

4. The method of Claim 3 wherein the benzotriazole has the formula:

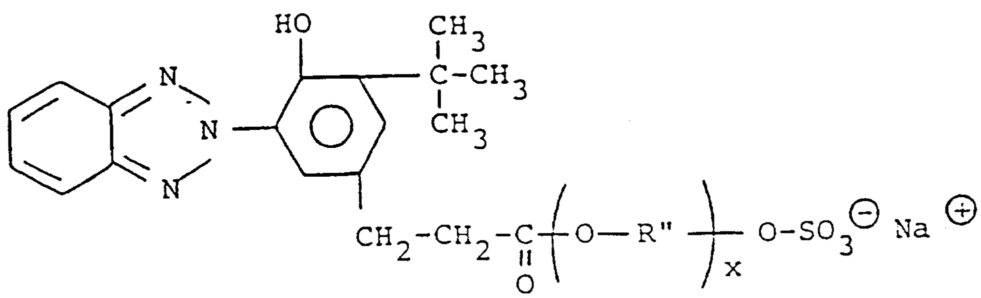

wherein x is 1 to 22 and R" is $-CH_2CH_2-$ or

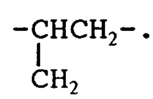

5. The method of Claim 3 wherein the material treated is film.

6. The method of Claim 3 wherein the material treated is fiber.

**Patentansprüche**

1. Sulfatiertes 2-(2'-Hydroxyaryl)-2H-Benzotriazol mit der Formel:

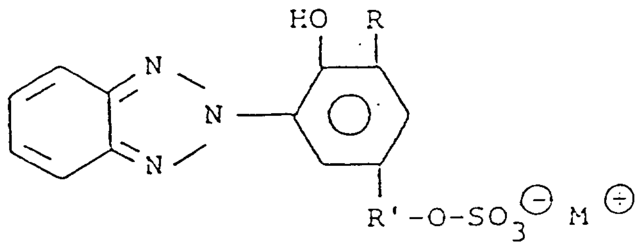

worin R eine sekundäre Alkyl- oder eine tertiäre Alkylgruppe mit bis zu 30 Kohlenstoffatomen ist, R' eine aliphatische Kette mit insgesamt 5 bis 69 Kohlenstoff- und Sauerstoffatomen in der Kette ist, und M Wasserstoff oder ein Metallion ist.

2. Benzotriazol nach Anspruch 1 mit der Formel:

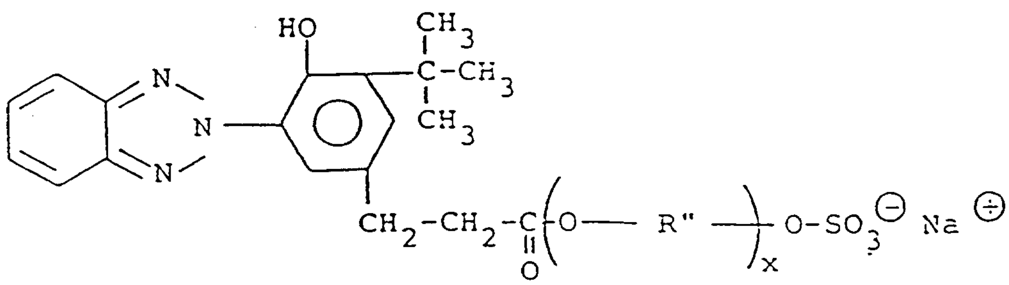

worin x gleich 1 bis 22 ist, und R'' ist $-CH_2CH_2-$ oder

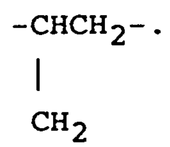

3. Verfahren zum Verbessern der Beständigkeit gegen Fleckanfärbung durch anionische Farbverbindungen und zum Verbessern der Lichtechtheit von Farben auf Nylonmaterial, welches die Behandlung der Faser mit einer wirksamen Menge einer wäßrigen Lösung eines sulfatierten 2-(2'-Hydroxyaryl)-2H-Benzotriazols bei milden Temperaturen mit der Formel umfaßt:

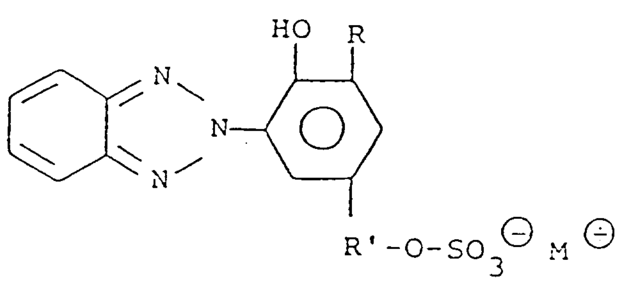

worin R eine sekundäre Alkyl- oder eine tertiäre Alkylgruppe mit bis zu 30 Kohlenstoffatomen ist, R' eine aliphatische Kette mit insgesamt 5 bis 69 Kohlenstoff- und Sauerstoffatomen in der Kette ist, und

M Wasserstoff oder ein Metallion ist.

4. Verfahren nach Anspruch 3, bei dem das Benzotriazol die folgende Formel besitzt:

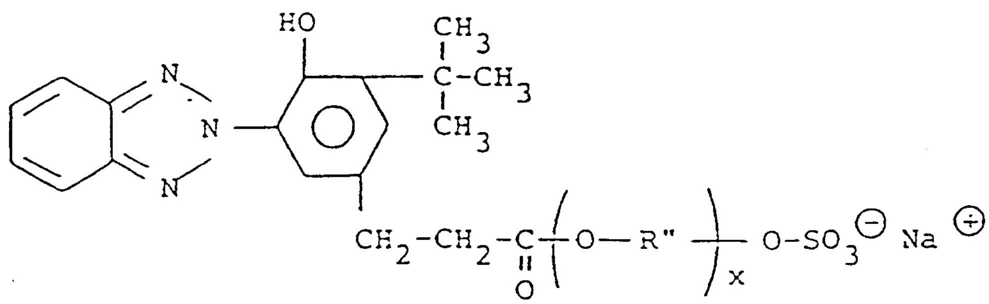

worin x gleich 1 bis 22 ist, und R" ist $-CH_2CH_2-$ oder

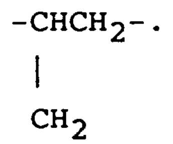

5. Verfahren nach Anspruch 3, bei dem das behandelte Material ein Film ist.

6. Verfahren nach Anspruch 3, bei dem das behandelte Material eine Faser ist.

## Revendications

1. Composé 2-(2'-hydroxyaryl)-2H-benzotriazole sulfaté ayant la formule

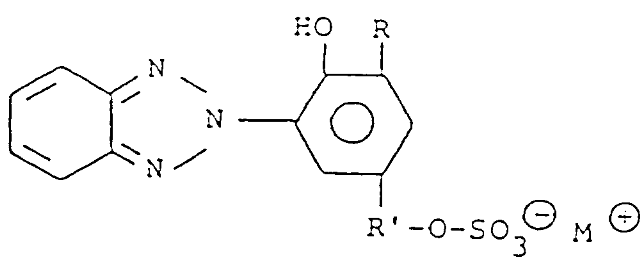

dans laquelle R est un groupe alkyle secondaire ou un groupe alkyle tertiaire comportant jusqu'à 30 atomes de carbone, R' est une chaîne aliphatique comportant un total de 5 à 69 atomes de carbone et d'oxygène dans la chaîne et dans laquelle M est un ion hydrogène ou un ion métallique.

2. Benzotriazole selon la revendication 1, caractérisé en ce qu'il est de formule :

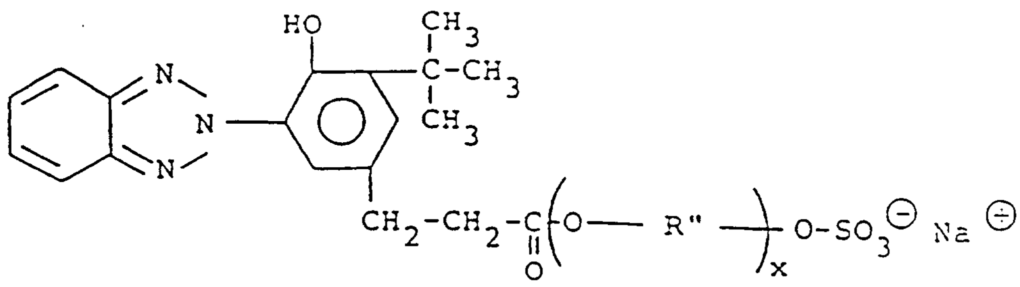

dans laquelle x est 1 à 22 et R" est $-CH_2CH_2-$ ou

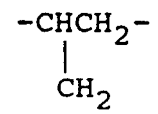

3. Méthode pour améliorer la résistance aux taches par des composés anioniques tachants et pour améliorer la stabilité à la lumière des teintures appliquées sur la matière de nylon qui comprend le traitement de la fibre à des températures modérées au moyen d'une quantité efficace d'une solution aqueuse de 2-(2'-hydroxyaryl)-2H-benzotriazole sulfaté ayant la formule :

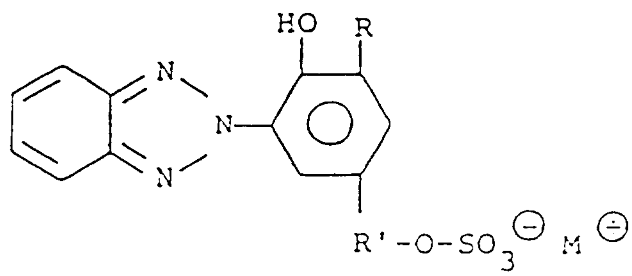

dans laquelle R est un groupe alkyle secondaire ou un groupe alkyle tertiaire comportant jusqu'à 30 atomes de carbone, R' est une chaîne aliphatique comportant un total de 5 à 69 atomes de carbone et d'oxygène dans la chaîne et dans laquelle M est un ion hydrogène ou un ion métallique.

4. Méthode selon la revendication 3, dans laquelle le benzotriazole est de formule :

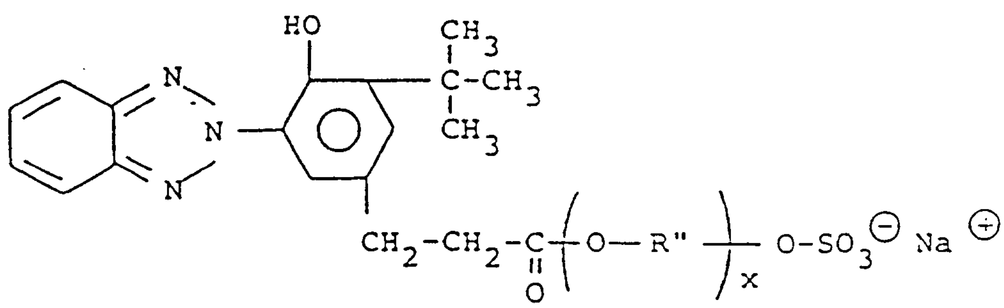

dans laquelle x est 1 à 22 et R" est $-CH_2CH2-$ ou

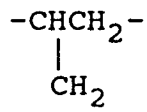

**5.** Méthode selon la revendication 3, dans laquelle la matière traitée est un film.

**6.** Méthode selon la revendication 3, dans laquelle la matière traitée est une fibre.